(19) Europäisches Patentamt
European Patent Office
Office européen des brevets

(11) Veröffentlichungsnummer : **0 023 891**
**B1**

(12) # EUROPÄISCHE PATENTSCHRIFT

(45) Veröffentlichungstag der Patentschrift : **14.09.83**

(51) Int. Cl.³ : **C 07 D213/64**, A 01 N 43/40

(21) Anmeldenummer : **80810241.2**

(22) Anmeldetag : **01.08.80**

(54) **Pyridyl-2-oxy-phenyloximäther und -ester, deren Herstellung und Verwendung als herbizide Mittel.**

(30) Priorität : **07.08.79 CH 7230/79**

(43) Veröffentlichungstag der Anmeldung : **11.02.81 Patentblatt 81/06**

(45) Bekanntmachung des Hinweises auf die Patenterteilung : **14.09.83 Patentblatt 83/37**

(84) Benannte Vertragsstaaten : **BE CH DE FR GB IT LI NL**

(56) Entgegenhaltungen :
**BE A 870 068**
**DE A 2 732 846**

**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Patentabteilung Postfach**
**CH-4002 Basel (CH)**

(72) Erfinder : **Pissiotas, Georg, Dr.**
**Breslauerstrasse 8**
**D-7850 Lörrach (DE)**
Erfinder : **Rempfler, Hermann, Dr.**
**Brückilsmattstrasse 16**
**CH-4107 Ettingen (CH)**

**EP 0 023 891 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Pyridyl-2-oxy-phenyloximäther und -ester, deren Herstellung und Verwendung als herbizide Mittel

Die vorliegende Erfindung betrifft neue Pyridyl-2-oxy-phenyloximäther und -ester, Verfahren zu deren Herstellung, sie als Wirkstoff enthaltende herbizide und das Pflanzenwachstum regulierende Mittel, sowie deren Verwendung als selektive Herbizide in Kulturpflanzenbeständen.

Aus dem Stand der Technik sind herbizid wirkende und pflanzenwuchsregulierende Pyridyloxyphenoxy-alkancarbonsäurederivate bekannt geworden, siehe beispielsweise die DOS No. 2 546 251, 2 649 706 oder 2 732 846. Kürzlich sind in der belgischen Patentschrift No. 870 068 Diphenyloxim-Verbindungen mit herbizider Wirkung beschrieben worden. Die Oximäther und Oximester vorliegender Erfindung sind neu.

Die erfindungsgemässen Pyridyl-2-oxy-phenyloximäther und -ester entsprechen der Formel I

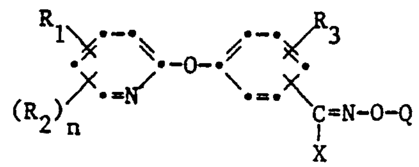

(I)

worin $R_1$ Wasserstoff, Halogen oder eine Gruppe Cyan, Nitro, Niederalkyl oder Halogenmethyl, $R_2$ Halogen, $R_3$ Wasserstoff, Halogen, die Nitro- oder die Cyanogruppe.

n 0, 1 oder 2, X Wasserstoff, Halogen oder eine der Gruppen Cyan, Nitro, $C_1$-$C_4$ Alkyl, $C_1$-$C_4$ Alkanoyl, Carbonsäure-$C_1$-$C_4$-alkylester oder Carbonsäureamid, Q ein $C_1$-$C_4$ Alkyl, das geradkettig oder verzweigt ist oder durch Heteroatome unterbrochen oder durch Halogen substituiert sein kann, ein $C_2$-$C_6$ Alkenyl oder Halogenalkenyl, mit bis zu 3 Halogenatomen ein $C_3$-$C_4$ Alkinyl, ein $C_2$-$C_4$ Cyanalkyl, eine $C_1$-$C_4$ Alkancarbonsäureester-Gruppe, eine $C_1$-$C_4$ Alkancarbonsäureamid-Gruppe, einen gegebenenfalls durch $R_2$ und $R_3$ substituierten Benzoylrest oder einen Carbamoylrest bedeuten.

In der Formel I ist unter Halogen, Fluor, Chlor, Brom oder Jod zu verstehen.

Der Ausdruck Alkyl allein oder als Teil eines Substituenten umfasst verzweigte oder unverzweigte $C_1$-$C_4$-Alkylgruppen. Beispiel sind Methyl, Aethyl, Propyl, Isopropyl, Butyl, Isobutyl, sec.-Butyl, tert.-Butyl. Sinngemäss enthalten Cyanalkyle ein und Alkancarbonsäureester mindestens zwei zusätzliche C-Atome.

Alkenyle bedeuten aliphatische Reste mit ein oder auch zwei Doppelbindungen (« Alkadienyle ») und maximal 6, bevorzugt 4 C-Atomen. Halogenalkenyle enthalten bis zu 3 Halogenatome, bevorzugt Chlor oder Brom. Alkinyl bedeutet Propinyl (Propargyl) und Butinyl.

Carbonsäureamide umfassen auch mono- oder symmetrisch oder unsymmetrisch di-substituierte Amide, wobei die Substituenten wahlweise $C_1$-$C_4$ alkyl, $C_2$-$C_6$ alkenyl, Propinyl oder Butinyl sowie einmal ein Phenylring sein können.

Beispiele für durch Heteroatome unterbrochene Alkyl-Ketten sind Methoxyäthyl, Aethoxyäthyl, Propoxyäthyl, Butoxyäthyl, Methoxypropyl, Aethylthioäthyl, Methylaminoäthyl, tert.-Butylaminoäthyl, Alkoxyalkoxyalkyl mit Methoxyäthoxyäthoxyäthyl.

Ein Carbamoylrest

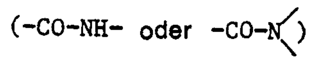

trägt am N-Atom ein oder zwei Reste aus der Gruppe $C_1$-$C_4$ Alkyl, $C_2$-$C_4$ Alkoxyalkyl, $C_2$-$C_6$ Alkenyl, $C_2$-$C_6$ Haloalkenyl, mit bis zu 3 Halogenatomen $C_3$-$C_4$ Alkinyl oder ein Wasserstoffatom und wahlweise einen Phenylring der unsubstituiert ist, oder, wie für $R_2$, $R_3$ angegeben, substituiert sein kann.

Bevorzugt sind solche Wirkstoffe der Formel I, in denen X die Methyl- oder Cyanogruppe darstellt und Q einen Esterrest

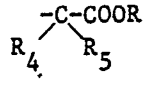

worin R $C_1$-$C_4$ alkyl, $R_4$ Wasserstoff oder $C_1$-$C_4$ Alkyl, $R_5$ Wasserstoff, $C_1$-$C_4$ Alkyl oder $C_2$-$C_4$ Alkoxy darstellt, und $R_1$ und $R_2$ sich in der 3- resp. 5-Stellung im Pyridylring befinden. Ferner ist $R_1$ vorzugsweise Cl oder $CF_3$ und $R_2$ Chlor.

Die Pyridyl-2-oxy-phenyloximäther und -ester der Formel I besitzen in Aufwandmengen von 0,5 bis 4 kg/ha und mehr eine ausgesprochen herbizide Wirkung insbesondere auf dikotyle Unkräuter, wie Sida, Sesbania, Amaranthus, Sinapis, Ipomoea, Galium, Pastinak, Rumex, Matricaria, Chrysanthemum, Abutilon, Solanum etc., einige wirken zudem bei höheren Aufwandmengen von mindestens 2 bis 4 kg/ha auf monokotyle Unkräuter wie Digitaria, Setaria und Echinochloa, während monokotyle Kulturpflanzen

wie Gerste, Weizen, Mais, Hafer und Reis bei niederen Aufwandmengen praktisch ungeschädigt bleiben und bei höheren Aufwandmengen nur schwach geschädigt werden.

Gute praktische Ergebnisse zur selektiven Bekämpfung von insbesondere dikotylen Unkräutern in Getreide, Mais und Reis konnten mit diesen Wirkstoffen erzielt werden.

Die Wirkstoffe bzw. die sie enthaltenden Mittel können pre-emergent auf angesäte Kulturflächen, aber vorzugsweise post-emergent auf verunkrautete Kulturpflanzenbestände als « Kontaktherbizide » appliziert werden.

Die Herstellung erfindungsgemässer Mittel erfolgt in an sich bekannter Weise durch inniges Vermischen und Vermahlen von Wirkstoffen der allgemeinen Formel I mit geeigneten Trägerstoffen und/oder Verteilungsmitteln, gegebenenfalls unter Zusatz von gegenüber den Wirkstoffen inerten Antischaum-, Netz-, Dispersions- und/oder Lösungsmitteln. Die Wirkstoffe können in den folgenden Aufarbeitungsformen vorliegen und angewendet werden :

Feste Aufarbeitungsformen : Stäubemittel, Streumittel, Granulate, Umhüllungsgranulate, Imprägniergranulate und Homogengranulate ;

In Wasser dispergierbare Wirkstoffkonzentrate : Spritzpulver (wettable powder), Pasten, Emulsionen, Emulsionskonzentrate.

Flüssige Aufbereitungsformen : Lösungen.

Die Herstellung der Verbindungen der Formel I erfolgt nach an sich bekannten Methoden.

Gemäss einer ersten Methode setzt man ein 2-Halogen-pyridin der Formel II

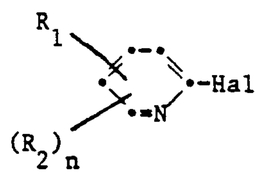

(II)

mit einem Hydroxybenzaldehyd der Formel III

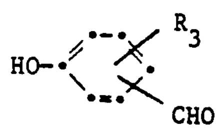

in Gegenwart einer Base um, wobei man ein Pyridyl-2-oxy-benzaldehyd der Formel IV erhält,

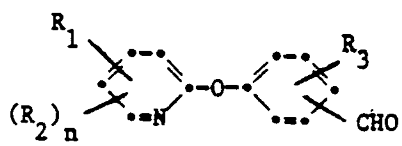

(IV)

welches mit Natriumborhydrid ($NaBH_4$) oder einem anderen milden Reduktionsmittel zum Pyridyl-2-oxy-benzylalkohol der Formel V reduziert wird,

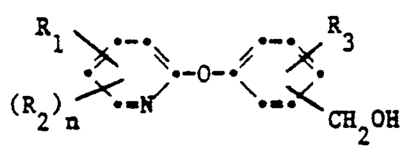

(V)

welche dann mittels Thionylchlorid, Thionylbromid oder Phosphoroxychlorid oder -bromid zum entsprechenden Pyridyl-2-oxy-benzylchlorid oder -bromid der Formel VI überführt wird.

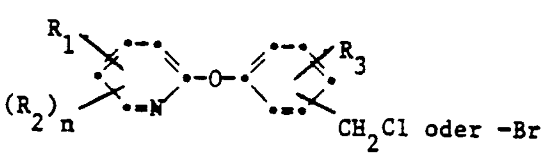

(VI)

Dieses wird mit Kalium- oder Natriumcyanid zur entsprechenden Cyanmethylverbindung der Formel VII umgesetzt,

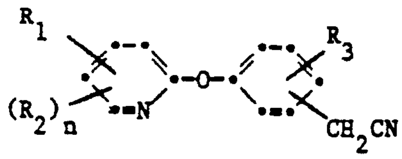

(VII)

welche dann mit Isoamylnitrit ($C_5H_{11}$ iso-ON = 0) in Gegenwart von Natriumäthylat ($NaOC_2H_5$) zum Pyridyl-2-oxy-phenyl-nitriloxim-salz der Formel VIII umgesetzt wird.

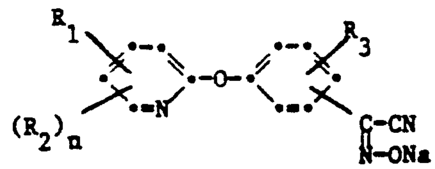

(VIII)

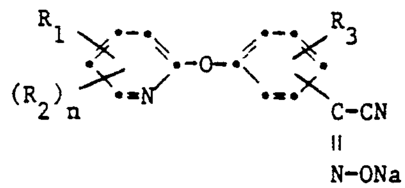

In den obigen Formeln II-VIII haben die Symbole n, $R_1$, $R_2$, und $R_3$ die weiter oben unter Formel I gegebene Bedeutung.

Ein Verfahren zur Herstellung der Pyridyl-2-oxy-phenyl-oximäther und -ester der Formel I, worin X Cyan bedeutet, ist dadurch gekennzeichnet, dass man ein entsprechend substituiertes Pyridyl-2-oxy-phenylnitriloxim-salz der obigen Formel VIII, worin, $R_1$, $R_2$ und $R_3$ die gegebene Bedeutung haben, in einem inerten, organischen Lösungsmittel in Gegenwart einer Base, mit einer Verbindung der Formel IX umsetzt,

$$Y—Q \qquad (IX)$$

worin Y ein Halogenatom oder einen abspaltbaren Säurrest bedeutet und Q die gegebene Bedeutung hat.

Eine weitere Methode um zu den Pyridyl-2-oxy-phenyl-oximäthern und -estern der Formel I zu gelangen besteht darin, dass man das 2-Halogenpyridin der Formel II mit einem entsprechend von $R_3$ substituierten Benzaldehyd oder Phenylketon umsetzt, in einem inerten organischen Lösungsmittel und in Gegenwart einer Base. Man erhält so entweder das Pyridyl-2-oxy-benzaldehyd der Formel IV oder ein entsprechendes Pyridyl-2-oxy-phenylketon der Formel X.

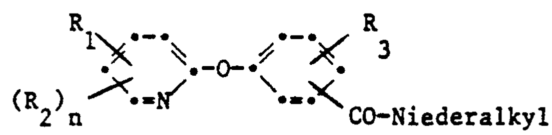

(X)

Wenn man diese mit Hydroxylamin ($H_2NOH$) umsetzt, erhält man ein Pyridyl-2-oxy-phenyloxim der Formel XI

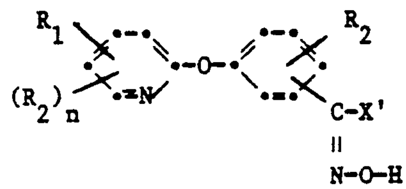

(XI)

worin X' Wasserstoff oder Niederalkyl bedeutet, während in den Formeln X und XI n, $R_1$, $R_2$ und $R_3$ die unter Formel I gegebene Bedeutung haben.

Ein weiteres Verfahren zur Herstellung der Pyridyl-2-oxy-phenyloximäther und -ester der Formel I ist dadurch gekennzeichnet, dass man ein Pyridyl-2-oxy-phenyloxim der obigen Formel XI, worin X' Wasserstoff oder Niederalkyl bedeuten und n, $R_1$, $R_2$ und $R_3$ die gegebene Bedeutung haben, in einem inerten, organischen Lösungsmittel, in Gegenwart einer Base, mit einer Verbindung der Formel IX umsetzt, worin Y ein Halogenatom oder einen abspaltbaren Säurerest bedeutet und Q die gegebene

Bedeutung hat und gegebenenfalls in Verbindungen der Formel I, in denen X Wasserstoff bedeutet, mit entsprechenden Reagentien, den Wasserstoff durch andere, unter die Definition von X fallende Reste austauscht.

Diese Umsetzungen werden bei Temperaturen zwischen 0 °C und 150 °C und in geeigneten Lösungsmitteln, wie Aceton, Methyläthylketon, Acetonitril, Dimethylformamid, Dimethylsulfoxid durchgeführt.

Als Basen eignen sich sowohl anorganische Basen wie Alkalimetallhydroxide, Alkali- und Erdalkalimetall-carbonate und -bicarbonate, aber auch trockener Ammoniak, sowie organische Basen, wie beispielsweise tertiäre Niederalkylamine, wie Triäthylamin, Trimethylamin oder auch cyclische Amine z. B. Pyridin, Collidin oder auch aromatische Amine wie Dimethylanilin.

Abspaltbare Säurereste Y sind z. B. eine Alkylsulfonylgruppe, eine Arylsulfonylgruppe, eine Nitrogruppe, oder ein halogenierter Fettsäurerest wie z. B. der Trichloressigsäureester.

Diese und andere Kondensationen von α-Oximino-Verbindungen und ihrer Alkalimetallsalze mit reaktionsfähigen Partnern Y—Q sind in « Organic Reactions » 1953, Band 7, Seite 343 und 373 beschrieben.

Oxime existieren immer in zwei stereoisomeren Formen, der syn- und anti-Form. Im Rahmen vorliegender Beschreibung sind beide stereoisomeren Formen für sich und als Gemische in beliebigem gegenseitigen Mischungsverhältnis zu verstehen.

Die nachfolgenden Beispiele beschreiben die Herstellung einiger Wirkstoffe der Formel I. Temperaturangaben beziehen sich auf Grad Celsius, Druckangaben sind in Millibar gegeben, Teile und Prozentangaben beziehen sich auf das Gewicht.

Beispiel 1

Herstellung von 4-(3',5'-Dichlorpyridyl-2-oxy)-phenylglyoxylnitril-2-oxim-methylcarbonyl-äth-1"-yl)-äther.

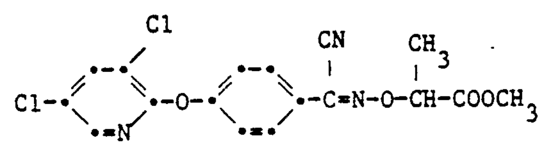

a) 61 g p-Hydroxybenzaldehyd, 81,2 g 2,3,5-Trichlorpyridin, 35 g pulverisiertes Kaliumhydroxid und 300 ml Dimethylsulfoxid wurden in einem Dreihalskolben 12 Stunden lang bei 150 °C gerührt. Nach dem Abkühlen wurde das Reaktionsgemisch in ein Eis/Wassergemisch gegossen, mit Essigester extrahiert, die Essigesterlösung über Natriumsulfat getrocknet und unter Vakuum eingedampft. Man erhielt so 112 g 4-(3',5'-Dichlorpyridyl-2-oxy)-benzaldehyd vom Schmelzpunkt 78 °C.

b) 13,4 g 4-(3',5'-Dichlorpyridyl-2'-oxy)-benzaldehyd wurden in 80 ml Methanol gelöst und bei einer Temperatur zwischen 25 °C und 30 °C mit 2 g Natriumborhydrid versetzt. Nach Beendigung der Reaktion wurde das Reaktionsgemisch 2 Stunden lang bei Raumtemperatur nachgerührt, ins Wasser gegossen, mit Essigester extrahiert und die organische Phase über Natriumsulfat getrocknet. Nach dem Eindampfen erhielt man 12,1 g 4-(3',5'-Dichlorpyridyl-2-oxy)-benzylalkohol als öliges Produkt mit dem Brechungsindex $n_D^{24}$ 1.613 3.

c) 11,3 g 4-(3',5'-Dichlorpyridyl-2'-oxy)-benzylalkohol wurden in 50 ml Toluol gelöst und bei Raumtemperatur mit 5 ml Thionylchlorid tropfenweise versetzt. Nach Beendigung der Gasentwicklung wurde das Reaktionsgemisch 2 Stunden bei 100 °C erwärmt. Nach Eindampfen unter Vakuum erhielt man 8 g 4-(3',5'-Pyridyl-2'-oxy)-benzylchlorid als festes Produkt, Schmelzpunkt 73-75 °C.

d) 26 g 4-(3',5'-Dichlorpyridyl-2'-oxy)-benzylchlorid, 6,5 g Kaliumcyanid und 50 ml Acetonitril wurden 20 Stunden lang unter Rückfluss erwärmt. Nach dem Abkühlen wurde das Reaktionsgemisch ins Wasser gegossen, die ausgefallene kristalline Substanz abgenutscht, mit Wasser gewaschen und im Vakuum getrocknet. Man erhielt so 24,5 g 4-(3',5'-Dichlorpyridyl-2'-oxy)-benzylcyanid vom Schmelzpunkt 107-108 °C.

e) In einem Dreihalskolben wurden unter Rühren 6 g Natriumäthylat in 80 ml absolutem Alkohol gelöst und auf 0 °C gekühlt. Bei dieser Temperatur wurden zuerst 24,2 g 4-(2',5'-Dichlorpyridyl-2'-oxy)-benzylcyanid dazugegeben und anschliessend bei Raumtemperatur 11,6 ml Isopentylnitrit zugetropft. Danach wurde das Reaktionsgemisch 24 Stunden bei Raumtemperatur weitergerührt und mit 200 ml Petroläther versetzt. Der ausgefallene Niederschlag wurde abfiltriert, mit Petroläther gewaschen und getrocknet. Man erhielt so 16,2 g 4-(3',5-Dichlorpyridyl-2'-oxy)-phenylglyoxylnitril-2-oxim-natriumsalz mit einem Schmelzpunkt von über 280 °C.

f) In einem Dreihalskolben wurden 12,1 g 4-(3',5'-Dichlorpyridyl-2'-oxy)-phenylglyoxylonitril-2-oxim-Natriumsalz in 50 ml Dimethylformamid gelöst. Hierauf wurden 4,2 ml 2-Brompropionsäuremethylester zugetropft. Das Reaktionsgemisch wurde über Nacht bei Raumtemperatur weitergerührt, und anschliessend in Wasser gegossen und mit Aether extrahiert. Nach dem Trocknen und Einengen des Aethers erhielt man 12,7 g der Titelverbindung als braunes Oel mit dem Brechungsindex $n_D^{23}$ 1.585 5. (Verbindung No. 8).

## Beispiel 2

Herstellung von 4-(3',5'-Dichlorpyridyl-2'-oxy)-acetophenyloxim-(methoxy-carbonyl-äth-1"-yl)-äther.

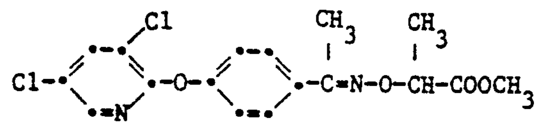

a) 27 g 4-(3',5'-Dichlorpyridyl-2'-oxy)-acetophenon (erhalten durch Umsetzung von 2,3,5-Trichlorpyridin mit para-Hydroxyacetophenon) wurden in 150 ml Alkohol gelöst und nach Zugabe von 100 ml Wasser, 6,6 g Hydroxylaminhydrochlorid und 9 g Natriumacetat, 4 Stunden lang bei 100 °C Badtemperatur gerührt. Nach dem Abkühlen wurde das ausgefallene kristalline Produkt abfiltriert und aus Alkohol umkristallisiert. Man erhält so 21 g 4-(3',5'-Dichlorpyridyl-2'-oxy)-acetophenyloxim vom Schmelzpunkt 118-119 °C.

b) 15 g des unter a) erhaltenen Oxims werden in einer Lösung aus 70 ml Methanol und 1,2 g Natrium gelöst und anschliessend unter Vakuum zur Trockene eingedampft. Das erhaltene Salz wurde in Dimethylformamid gelöst und mit 8,5 g 2-Brompropionsäuremethylester bei Raumtemperatur tropfenweise versetzt. Nach 12 Stunden Rühren wurde das Reaktionsgemisch ins Wasser gegossen, mit Essigester extrahiert, die Lösung über Natriumsulfat getrocknet und unter Vakuum eingedampft. Man erhielt so 19 g der Titelverbindung als Oel mit dem Brechungsindex $n_D^{23}$ 1,573 4. (Verbindung No. 1).

In analoger Weise zu diesen Beispielen werden folgende Verbindungen hergestellt :

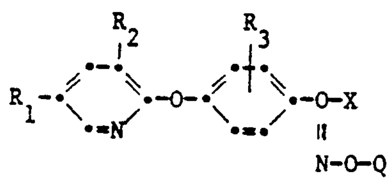

Tabelle 1

| Verbg. No. | $R_1$ | $R_2$ | $R_3$ | X | Q | physikalische Daten |
|---|---|---|---|---|---|---|
| 1 | Cl | Cl | H | $CH_3$ | $CH(CH_3)COOCH_3$ | $n_D^{23}$ 1.5734 |
| 2 | Cl | Cl | H | $CH_3$ | $CONHCH_3$ | Harz |
| 3 | Cl | Cl | H | $CH_3$ | $CONHC_6H_5$ | Smp. 113° |
| 4 | Cl | Cl | H | $CH_3$ | $CONHC_6H_3$(3,4-Cl) | Harz |
| 5 | Cl | Cl | H | $CH_3$ | $COC_6H_5$ | Smp. 114° |
| 6 | Cl | Cl | H | $CH_3$ | $CH_2CN$ | $n_D^{23}$ 1.5788 |
| 7 | Cl | Cl | H | $CH_3$ | $COCH_3$ | Smp. 115-6° |
| 8 | Cl | Cl | H | CN | $CH(CH_3)COOCH_3$ | $n_D^{23}$ 1.5855 |
| 9 | $CF_3$ | Cl | H | CN | $CH(CH_3)COOCH_3$ | $n_D^{30}$ 1.5354 |
| 10 | $CF_3$ | Cl | H | CN | $CH(CH_3)COOC_2H_5$ | |
| 11 | $CF_3$ | Cl | H | CN | $CH(CH_3)CN$ | |

(Fortsetzung)

| Verbg. No. | $R_1$ | $R_2$ | $R_3$ | X | Q | physikalische Daten |
|---|---|---|---|---|---|---|
| 12 | $CF_3$ | Cl | H | CN | $CH(CH_3)COOC_3H_7$iso | |
| 13 | $CF_3$ | Cl | H | CN | $CH(CH_3)COOC_3H_7$n | |
| 14 | $CF_3$ | Cl | H | CN | $CH(CH_3)COOC_4H_9$iso | $n_D^{30}$ 1.5136 |
| 15 | $CF_3$ | Cl | H | CN | $CONHCH_3$ | |
| 16 | $CF_3$ | Cl | H | CN | $CONHC_6H_5$ | |
| 17 | $CF_3$ | Cl | H | CN | $COCH_3$ | |
| 18 | $CF_3$ | Cl | H | CN | $COC_6H_4$(4'Cl) | |
| 19 | $CF_3$ | Cl | H | CN | $CO(CH_3)COOC_4H_9$n | |
| 20 | $CF_3$ | H | H | CN | $CH(CH_3)COOCH_3$ | |
| 21 | $CClF_2$ | Cl | H | CN | $CH(CH_3)COOCH_3$ | |

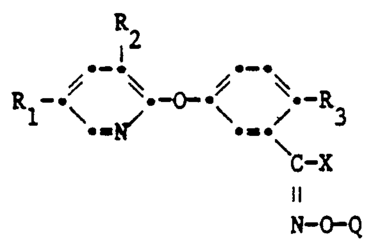

Tabelle 2

| Verbg. No. | $R_1$ | $R_2$ | $R_3$ | X | Q | physikalische Daten |
|---|---|---|---|---|---|---|
| 1 | Cl | Cl | Cl | CN | $CH(CH_3)COOCH_3$ | |
| 2 | $CF_3$ | Cl | Cl | CN | $CH(CH_3)COOCH_3$ | |
| 3 | Cl | Cl | H | $CH_3$ | $CH(CH_3)COOCH_3$ | $n_D^{24}$ 1.5759 |
| 4 | $CF_3$ | Cl | Cl | $CH_3$ | $CH(CH_3)COOCH_3$ | |
| 5 | Cl | Cl | Cl | $CH_3$ | $COCH_3$ | |
| 6 | $CF_3$ | Cl | Cl | $CH_3$ | $COCH_3$ | |
| 7 | Cl | Cl | Cl | $CH_3$ | $COC_6H_5$ | |
| 8 | $CF_3$ | Cl | Cl | $CH_3$ | $COC_6H_5$ | |
| 9 | Cl | Cl | Cl | $CH_3$ | $CONHCH_3$ | |
| 10 | $CF_3$ | Cl | Cl | $CH_3$ | $CONHCH_3$ | |
| 11 | Cl | Cl | Cl | $CH_3$ | $CONHC_6H_5$ | |
| 12 | Cl | Cl | Cl | $CH_3$ | $CONHC_6H_5$ | |
| 13 | Cl | Cl | $NO_2$ | $CH_3$ | $COOCH_3$ | |

Beispiel 14

Herstellung von gebrauchsbereiten Anwendungsformen und Wirkstoffkonzentraten.

Granulat

Zur Herstellung eines 5 %igen Granulates werden die folgenden Stoffe verwendet :

5 Teile 4-(3',5'-Dichlorpyridyl-2'-oxy)-acetophenoxim-(methoxy-carbonyl-äth-1-yl)-äther
0,25 Teile epoxidiertes Pflanzenöl
0,25 Teile Cetylpolyglykoläther,
3,50 Teile Polyäthylenglykol,
91 Teile Kaolin (Korngrösse : 0,3-0,8 mm).

Die Aktivsubstanz wird mit Epichlorhydrin vermischt und in 6 Teilen Aceton gelöst, hierauf wird Polyäthylenglykol und Cetylpolyglykoläther zugesetzt. Die so erhaltene Lösung wird auf Kaolin aufgesprüht und anschliessend im Vakuum verdampft.

Spritzpulver

Zur Herstellung eines a) 70 %igen und b) 10 %igen Spritzpulvers werden folgende Bestandteile verwendet :

a) 70 Teile 4-(3',5'-Dichlorpyridyl-2-oxy)-phenylglyoxylnitril-2-oxim-methyl-äth-1"-yl)-äther
5 Teile Natriumdibutylnaphthylsulfonat,
3 Teile Naphthalinsulfonsäuren-Phenolsulfonsäuren-Formaldehyd-Kondensat 3 : 2 : 1,
10 Teile Kaolin,
12 Teile Champagne-Kreide ;

b) 10 Teile des obigen Wirkstoffs,
3 Teile Gemisch der Natriumsalze von gesättigten Fettalkoholsulfaten
5 Teile Naphthalinsulfonsäuren-Formaldehyd-Kondensat
82 Teile Kaolin.

Der verwendete Wirkstoff wird auf die entsprechenden Trägerstoffe (Kaolin und Kreide) aufgezogen und anschliessend vermischt und vermahlen. Man erhält Spritzpulver von vorzüglicher Benetzbarkeit und Schwebefähigkeit. Aus solchen Spritzpulvern können durch Verdünnung mit Wasser Suspensionen von 0,1-8 % Wirkstoff erhalten werden, die sich zur Unkrautbekämpfung in Pflanzenkulturen eignen.

Paste

Zur Herstellung einer 45 %igen Paste werden folgende Stoffe verwendet :

45 Teile 4-(3',5'-Dichlorpyridyl-2'-oxy)-phenylglyoxynitril-2-oxim-methyl-carbonyl-äth-1"-yl)-äther.
5 Teile Natriumaluminiumsilikat,
14 Teile Cetylpolyglykoläther mit 8 Mol Aethylenoxid,
1 Teil Oleylpolyglykoläther mit 5 Mol Aethylenoxid,
2 Teile Spindelöl
10 Teile Polyäthylenglykol,
23 Teile Wasser.

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

Emulsionskonzentrat

Zur Herstellung eines 25 %igen Emulsionskonzentrates werden

25 Teile 4-(3',5'-Dichlorpyridyl-2'-oxy)-acetophenyloxim-(methoxy-carbonyl-äth-1"-yl)-äther
5 Teile einer Mischung von Nonylphenolpolyoxyäthylen oder Calciumdodecylbenzolsulfat,
15 Teile Cyclohexanon,
55 Teile Xylol

miteinander vermischt. Dieses Konzentrat kann mit Wasser zu Emulsionen auf geeignete Konzentrationen von z. B. 0,1 bis 10 % verdünnt werden.

Beispiel 3

Ermittlung der herbiziden im Vor- und Nachauflaufverfahren.

Pre-emergente Herbizid-Wirkung (Keimhemmung)

Im Gewachshaus wird unmittelbar nach der Einsaat der Versuchspflanzen in Saatschalen die Erdoberfläche mit einer wässerigen Dispersion der Wirkstoffe, erhalten aus einem 25 %igen Emulsionskonzentrat resp. aus einem 25 %igen Spritzpulver mit Wirkstoffen, die wegen ungenügender Löslichkeit nicht als Emulsionskonzentrat hergestellt werden können, behandelt. Es wurden vier verschiedene Konzentrationsreihen angewendet, entsprechend 4, 2, 1 und 0,5 kg Wirksubstanz pro Hektar. Die Saatschalen werden im Gewächshaus bei 22-25 °C und 50-70 % rel. Luftfeuchtigkeit gehalten und der Versuch nach 3 Wochen ausgewertet. Die Verbindungen No. 8, 9 und 14 der Tabelle 1 zeigten noch starke Wirkung, vor allem gegen dikotyl Pflanzen bei Aufwandmengen von 0.5 kg pro Hektar.

Post-emergente Herbizid-Wirkung (Kontaktherbizid)

Eine grössere Anzahl Unkräuter und Kulturpflanzen, sowohl monocotyle wie dicotyle, wurden nach dem Auflaufen (im 4- bis 6-Blattstadium) mit einer wässerigen Wirkstoffdispersion in Dosierungen von 0,125 ; 0,25 ; 0,5 kg Wirksubstanz pro Hektar auf die Pflanze gespritzt und diese bei 24°-26 °C und 45-60 % rel. Luftfeuchtigkeit gehalten. Mindestens 15 Tage nach Behandlung wird der Versuch ausgewertet. Die Verbindungen No. 8, 9 und 14 der Tabelle 1 zeigten noch starke Wirkung, vor allem gegen dikotyle Pflanzen bei einer Aufwandmenge von 0,25 kg pro Hektar.

**Ansprüche**

1. Pyridyl-2-oxy-phenyloximäther und -ester der Formel I

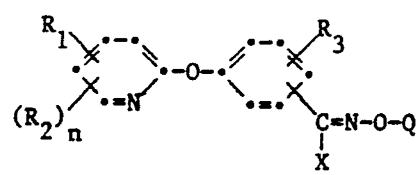

(I)

worin $R_1$ Wasserstoff, Halogen oder eine der Gruppen Cyan, Nitro, $C_1$-$C_4$ Alkyl oder Halogenmethyl, $R_2$ Halogen, $R_3$ Wasserstoff, Halogen oder die Nitro- oder Cyanogruppe, n 0, 1 oder 2, X Wasserstoff, Halogen oder eine der Gruppen Cyan, Nitro, $C_1$-$C_4$ Alkyl, $C_1$-$C_4$-Alkanoyl, Carbonsäure-$C_1$-$C_4$-alkylester oder Carbonsäureamid, Q ein $C_1$-$C_4$ Alkyl, das geradkettig oder verzweigt ist oder durch Heteroatome unterbrochen oder durch Halogen substituiert sein kann, ein $C_2$-$C_8$ Alkenyl oder Halogenalkenyl, mit bis zu 3 Halogenatomen ein $C_3$-$C_4$ Alkinyl, ein $C_2$-$C_4$ Cyanalkyl, eine $C_1$-$C_4$ Alkancarbonsäureester-Gruppe, eine $C_1$-$C_4$ Alkancarbonsäure-Amidgruppe, einen gegebenenfalls durch $R_2$ und $R_3$ substituierten Benzoylrest oder einen Carbamoylrest bedeuten.

2. Die Pyridyl-2-oxy-phenyl-oximäther und -ester der Formel I, Anspruch 1, worin X die Methyl- oder Cyanogruppe bedeutet.

3. Die Pyridyl-2-oxy-phenyl-oximäther und -ester der Formel I, Anspruch 1 welche der Formel

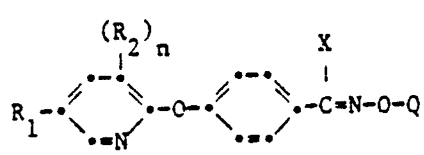

entsprechen, worin n, Q, $R_1$, $R_2$ und X die unter Formel I, Anspruch 1 gegebene Bedeutung haben.

4. 4-(3',5'-Dichlorpyridyl-2'-oxy)-phenylglyoxylnitril-2-oxim-(methoxycarbonyl-äth-1''-yl)-äther gemäss Anspruch 1.

5. 4-(3',5'-Dichlorpyridyl-2'-oxy)-acetophenyloxim-(methoxy-carbonyl-äth-1''-yl)-äther, gemäss Anspruch 1.

6. Verfahren zur Herstellung der Pyridyl-2-oxy-phenyloximäther und -ester der Formel I, Anspruch 1 in denen X Cyan bedeutet, dadurch gekennzeichnet, dass man ein Pyridyl-2-oxy-phenynitriloximsalz der Formel VIII

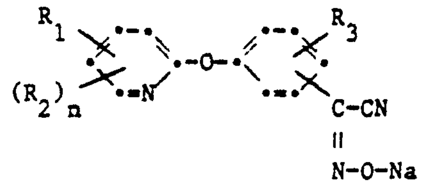

(VIII)

worin n, $R_1$, $R_2$ und $R_3$ die unter Formel I, Anspruch I gegebene Bedeutung haben, in einem inerten, organischen Lösungsmittel, in Gegenwart einer Base, mit einer Verbindung der Formel IX umsetzt

Y—Q (IX)

worin Y ein Halogenatom oder einen abspaltbaren Säurerest bedeutet und Q die unter Formel I, Anspruch 1 gegebene Bedeutung hat.

7. Verfahren zur Herstellung der Pyridyl-2-oxy-phenyloximäther und -ester der Formel I, Anspruch 1, dadurch gekennzeichnet, dass man ein Pyridyl-2-oxy-phenyloximäther und -ester der Formel XI

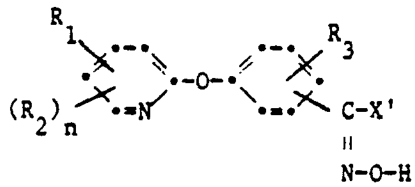

(XI)

worin X' Wasserstoff oder Niederalkyl bedeutet und n, $R_1$, $R_2$ und $R_3$ die unter Formel I, Anspruch 1 gegebene Bedeutung haben, in einem inerten organischen Lösungsmittel und in Gegenwart einer Base, mit einer Verbindung der Formel IX umsetzt,

Y—Q (IX)

worin Y ein Halogenatom oder ein abspaltbarer Säurerest bedeutet und Q die unter Formel I, Anspruch 1 gegebene Bedeutung haben und gegebenenfalls in Verbindungen der Formel I, in denen X durch Wasserstoff verkörpert ist, diesen Rest mit einem geeigneten Reagens durch einen anderen in der Definition von X gegebenen Rest auswechselt.

8. Ein herbizides Mittel, dadurch gekennzeichnet, dass es als Wirkstoff mindestens einen Oximäther oder Oximester der Formel I, Anspruch 1, enthält.

9. Die Verwendung der Verbindungen der Formel I, Anspruch 1, zur selektiven Bekämpfung von Unkräutern in Kulturpflanzenbeständen.

**Claims**

1. A pyridyl-2-oxy-phenyloxime ether or ester of the formula I

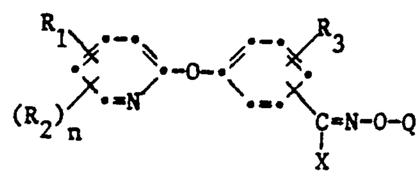

(I)

wherein $R_1$ is hydrogen, halogen or a cyano, nitro, $C_1$-$C_4$-alkyl or halomethyl group, $R_2$ is halogen, $R_3$ is hydrogen, halogen, the nitro or cyano group, n is 0, 1 or 2, X is hydrogen, halogen or a member selected from the group consisting of cyano, nitro, $C_1$-$C_4$ alkyl, $C_1$-$C_4$ alkanoyl, carboxylic acid $C_1$-$C_4$ alkyl ester or carbamoyl, Q is $C_1$-$C_4$-alkyl which is straight-chain or branched or which can be interrupted by heteroatoms or substituted by halogen ; or is $C_2$-$C_6$ alkenyl or $C_1$-$C_3$ haloalkenyl ; $C_3$-$C_4$ alkynyl ; or is $C_2$-$C_4$ cyanoalkyl ; a $C_1$-$C_4$ alkanecarboxylic acid ester group ; a $C_1$-$C_4$ alkanecarboxylic acid amide group ; a benzoyl radical which is unsubstituted or substituted by $R_2$ and $R_3$, or a carbamoyl radical.

2. A pyridyl-2-oxy-phenyloxime ether or ester of the formula I according to claim 1, wherein X is the methyl or cyano group.

3. A pyridyl-2-oxy-phenyloxime ether or ester of the formula I according to claim 1 of the formula

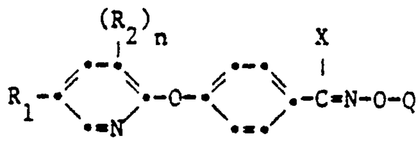

wherein n, Q, $R_1$, $R_2$ and X are as defined for formula I in claim 1.

4. 4-(3′,5′-Dichloropyridyl-2′-oxy)-phenylglyoxylonitrile-2-oxime-(methoxycarbonyl-eth-1″-yl)ether according to claim 1.

5. 4-(3′,5′-Dichloropyridyl-2′-oxy)-acetophenyloxime-(methoxycarbonyl-eth-1″-yl)ether according to claim 1.

6. A process for the production of a pyridyl-2-oxy-phenyl-oxime ether or ester of the formula I according to claim 1, wherein X is cyano, which process comprises reacting a pyridyl-2-oxy-phenylnitrile oxime salt of the formula VIII

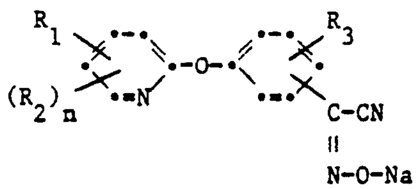

(VIII)

wherein n, $R_1$, $R_2$ and $R_3$ are as defined for formula I in claim 1, in an inert organic solvent and in the presence of a base, with a compound of the formula IX

$$Y—Q \qquad (IX)$$

wherein Y is a halogen atom or a removable acid radical and Q is as defined for formula I in claim 1.

7. A process for the production of a pyridyl-2-oxy-phenyl-oxime ether or ester of the formula I according to claim 1, which process comprises reacting a pyridyl-2-oxy-phenyloxime ether or ester of the formula XI

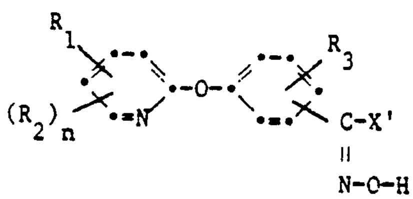

(XI)

wherein X′ is hydrogen or lower alkyl, and n, $R_1$, $R_2$ and $R_3$ are as defined for formula I in claim 1, in an inert organic solvent and in the presence of a base, with a compound of the formula IX

$$Y—Q \qquad (IX)$$

wherein Y is a halogen atom or a removable acid radical, and Q is as defined for formula I in claim 1, and, if desired, in a compound of the formula I, in which X is hydrogen, replacing hydrogen by another radical falling within the definition of X by means of a suitable reagent.

8. A herbicidal composition which contains, as active ingredient, at least one oxime ether or ester of the formula I according to claim 1.

9. A method of selectively controlling weeds in crops of cultivated plants, which comprises applying to said crops a herbicidally effective amount of a compound of the formula I according to claim 1.

**Revendications**

1. Pyridyl-2-oxy-phényloximéthers et -esters de formule I

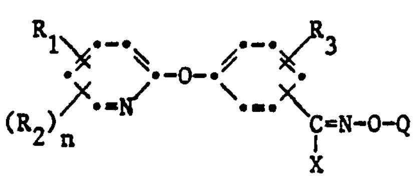

(I)

où $R_1$ représente un hydrogène, un halogène ou l'un des groupes cyano, nitro, alcoyle en $C_1$ à $C_4$ ou halogénométhyle, $R_2$ représente un halogène, $R_3$ représente un hydrogène, un halogène ou le groupe nitro ou cyano, n vaut 0, 1 ou 2, X représente un hydrogène, un halogène ou l'un des groupes cyano, nitro, alcoyle en $C_1$ à $C_4$, alcanoyle en $C_1$ à $C_4$, alcoyle en $C_1$ à $C_4$-ester d'acide carboxylique ou amide d'acide carboxylique, Q représente un alcoyle en $C_1$ à $C_4$, qui est à chaîne droite ou ramifiée ou peut être interrompu par des hétéroatomes ou substitué par des halogènes, un alcényle en $C_2$ à $C_6$ ou un halogénalcényle ayant jusqu'à 3 atomes d'halogène, un alcynyle en $C_3$ à $C_4$, un cyanalcoyle en $C_2$ à $C_4$, un groupe ester d'acide alcane en $C_1$ à $C_4$-carboxylique, un groupe amide d'acide alcane en $C_1$ à $C_4$-carboxylique, un radical benzoyle éventuellement substitué par $R_2$ et $R_3$ ou un radical carbamoyle.

2. Les pyridyl-2-oxy-phényl-oximéthers et -esters de formule I de la revendication 1 où X représente le groupe méthyle ou le groupe cyano.

3. Les pyridyl-2-oxy-phényl-oximéthers et -esters de formule I de la revendication 1 qui correspondent à la formule

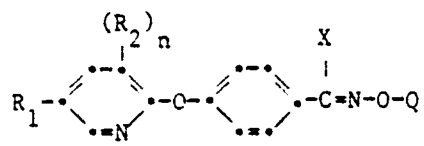

où n, Q, $R_1$, $R_2$ et X ont la signification donnée pour la formule I de la revendication 1.

4. 4-(3',5'-dichloropyridyl-2'-oxy)-phénylglyoxynitrile-2-oxime (méthoxycarbonyl-éth-1''-yl)-éther selon la revendication 1.

5. 4-(3',5'-dichloropyridyl-2'-oxy)-acétophényloxime-(méthoxy-carbonyl-éth-1''-yl)-éther selon la revendication 1.

6. Procédé de préparation des pyridyl-2-oxy-phényloxime-éthers et -esters de formule I de la revendication 1 où X représente un cyano, caractérisé en ce qu'on fait réagir un sel de pyridyl-2-oxy-phénylnitriloxime de formule VIII

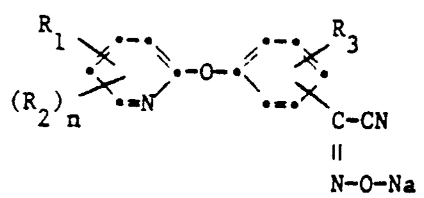

(VIII)

où n, $R_1$, $R_2$ et $R_3$ ont la signification donnée pour la formule I de la revendication 1, dans un solvant organique inerte, en présence d'une base, avec un composé de formule IX

$$Y—Q \qquad (IX)$$

où Y représente un atome d'halogène ou un radical acide éliminable et Q a la signification donnée pour la formule I de la revendication 1.

7. Procédé de préparation des pyridyl-2-oxy-phényloxime-éthers et -esters de formule I de la revendication 1, caractérisé en ce qu'on fait réagir un pyridyl-2-oxy-phényloximéther et -ester de formule XI

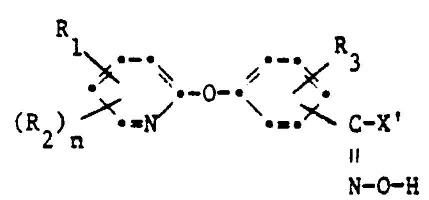

(XI)

où X' représente un hydrogène ou un alcoyle inférieur et où n, $R_1$, $R_2$ et $R_3$ ont la signification donnée pour la formule I de la revendication 1, dans un solvant organique inerte et en présence d'une base, avec un composé de formule IX

$$Y—Q \qquad (IX)$$

où Y représente un atome d'halogène ou un radical acide séparable et Q a la signification donnée pour la formule I de la revendication 1, où X est incarné par l'atome d'hydrogène, et en ce qu'on échange ce

radical avec un réactif approprié contre un autre radical donné dans la définition de X.

8. Agent herbicide caractérisé en ce qu'il contient comme matière active au moins un oximéther ou oximester de formule I de la revendication 1.

9. Application des composés de formule I de la revendication 1 à la lutte sélective contre les mauvaises herbes dans les cultures.